(19) Europäisches Patentamt
European Patent Office
Office européen des brevets

(11) Veröffentlichungsnummer: **0 013 900**
**B1**

(12) # EUROPÄISCHE PATENTSCHRIFT

(45) Veröffentlichungstag der Patentschrift: **01.12.82**

(51) Int. Cl.³: **C 07 D 265/26**

(21) Anmeldenummer: **80100086.0**

(22) Anmeldetag: **09.01.80**

(54) **Verfahren zur kontinuierlichen Herstellung von Isatosäureanhydrid.**

(30) Priorität: **26.01.79 DE 2902978**

(43) Veröffentlichungstag der Anmeldung: **06.08.80 Patentblatt 80/16**

(45) Bekanntmachung des Hinweises auf die Patenterteilung: **01.12.82 Patentblatt 82/48**

(84) Benannte Vertragsstaaten: **BE CH DE FR GB NL**

(56) Entgegenhaltungen:
**DE-A-2 258 150**
**DE-B-1 287 580**
**GB-A-1 436 810**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Klipper, Gerhard, Dr., Waldstrasse 3, D-6719 Battenberg (DE)**
Erfinder: **Grimmer, Johannes, Duererstrasse 12, D-6700 Ludwigshafen (DE)**
Erfinder: **Tonne, Peter, Dr., Triftbrunnenweg 63, D-6730 Neustadt 1 (DE)**
Erfinder: **Horn, Hans, Christopf, Dr., Auf der Au 5, D-6715 Lambsheim (DE)**
Erfinder: **Hetzel, Eckhard, Sandweg 26, D-6712 Bobenheim-Roxheim (DE)**

**EP 0 013 900 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur kontinuierlichen Herstellung von Isatosäureanhydride

Die Erfindung betrifft ein Verfahren zur kontinuierlichen Herstellung von Isatosäureanhydriden durch zweistufige Umsetzung von phthalamidsaurem und/oder phthalimidsaurem Alkali mit Alkalihypohalogeniten, indem die erste Stufe weitgehend adiabatisch und beide Stufen unter Verwendung hoher Strömungsgeschwindigkeiten durchgeführt werden, wobei zunächst Phthalimid und/oder Phthalamidsäure in einer bestimmten Alkalilaugenmenge gelöst werden, die Lösung mit Hypohalogeniten vermischt und bei bestimmter Temperatur umgesetzt wird, schliesslich Säure zugegeben und die Reaktion bei bestimmter Temperatur zu Ende geführt wird.

Aus den D-AS Nrn. 1950281 und 2000698 ist ein Verfahren zur kontinuierlichen Herstellung von Anthranilsäure durch Umsetzung von phthalamidsaurem und/oder phthalimidsaurem Alkali mit Hypohalogeniten in wässrigem Medium bekannt, wobei man

a) eine wässrige Lösung von phthalamidsaurem und/oder phthalimidsaurem Alkali und eine wässrige Lösung von Alkalihypochlorit in einer Mischvorrichtung mischt,

b) die erhaltene Mischung im ersten Teil eines engen Reaktionsrohres mit hoher Strömungsgeschwindigkeit bei 10 bis 50°C unter weitgehend adiabatischen Bedingungen umsetzt, anschliessend

c) die aus dem ersten Teil des Reaktionsrohres mit hoher Strömungsgeschwindigkeit abströmende Umsetzungsmischung im zweiten Teil des genannten Rohres bei 60 bis 80°C zu Ende umsetzt, und

d) aus der abströmenden alkalischen Umsetzungsmischung in üblicher Weise Anthranilsäure und/oder Isatosäureanhydrid abtrennt.

Bei diesem Verfahren wird nur ein Teil des freien Alkalihydroxids schon beim Lösen des Ausgangsstoffes verwendet, ein weiterer Teil der Hypochloritlösung zugesetzt. Vorteilhaft gelangen wässrige Lösungen von 10 bis 50 Gewichtsprozent Phthalimid und/oder Phthalamid zur Anwendung, die von 1 bis 1,1 mol Alkalihydroxid je Mol Phthalimid/Phthalamid enthalten. Es wird angegeben, dass die wässrigen Hypohalogenitlösungen vorteilhaft von 8 bis 15 Gewichtsprozent Hypohalogenit und von 0 bis 3, vorzugsweise von 0,02 bis 2,1 mol Alkalihydroxid je Mol Phthalimid/Phthalamidsäure enthalten. In Beispiel 1 (Herstellung von Anthranilsäure) befindet sich Alkalihydroxid sowohl in der Phthalimidlösung, in einer Menge von 1,1 mol NaOH je Mol Phthalimid, als auch in der Hypochloritlösung (1,4 mol NaOH, bezogen auf 1 mol Phthalimid).

Die D-AS Nr. 1950281 gibt an, dass durch Einstellung der Ausgangslösungen in bezug auf Alkalikonzentration die Bildung des Endstoffes beeinflusst wird. Bei einer Menge von 0,9 bis 1,1 mol Alkali je Mol Phthalimid bzw. Phthalamidsäure im Ausgangsgemisch erhält man Isatosäureanhydrid. Nur für diesen Fall sieht die Auslegeschrift, wie Beispiel 2 zeigt, eine Zugabe des gesamten Alkali zum Ausgangsstoff vor. Lediglich bei der Herstellung von Anthranilsäure, aber nicht im Falle der Isatosäureanhydridherstellung, wird gegebenenfalls während der Stufe b und/oder der Stufe c ein Reduktionsmittel zugesetzt, wie die deutsche Auslegeschrift Nr. 2000698 im Vergleich zu der deutschen Auslegeschrift Nr. 1950281 zeigt. Unter den für die Anthranilsäureherstellung verwendeten Reduktionsmitteln wird zwar schweflige Säure genannt, aber, wie das Beispiel und die Beschreibung der D-AS Nr. 2000698 zeigen, Natriumsulfit und Natriumbisulfit bevorzugt verwendet. Bezüglich Verweilzeit der Reaktion vor Zugabe des Reduktionsmittels und pH-Wert nach der Zugabe werden keine spezifischen Werte genannt. Wie die D-AS Nrn. 2000698 und 1950281 (Spalte 4, Zeilen 56 bis 60) zeigen, fällt das Reaktionsgemisch am Ende der Reaktion als alkalische Lösung an. Erst bei der Aufarbeitung der anthranilsäure wird Säure zur Fällung des Endstoffs zugesetzt. Im Falle der Herstellung von Isatosäureanhydrid (Beispiel 2 der D-AS Nr. 1950281) wird kein Reduktionsmittel zugesetzt und das Reaktionsgemisch erst nach der Reaktion bei der Aufarbeitung durch Säurezugabe auf pH 7 eingestellt und der so ausgefällte Endstoff abgetrennt. Das Verfahren liefert Isatosäureanhydrid in unbefriedigender Ausbeute, Reinheit und Raum-Zeit-Ausbeute.

Es ist aus DRP Nr. 127138 bekannt, dass Phthalimid in alkalischer Lösung mit Hypohalogeniten zu Isatosäureanhydrid umgesetzt werden kann. Die bei der Reaktion auftretenden Schwierigkeiten, insbesondere die unbefriedigende Ausbeute und Reinheit des Endstoffs, werden von E. Mohr [„J. pr. Chemie" (2), Bd. 80, S. 1-33 (1909)] beschrieben. Ein störungsfreier Betrieb gerade im grosstechnischen Massstab ist bei diesem Verfahren nicht möglich.

In dem erfindungsgemässen Verfahren aus nächsten Kommenden Stande der Technik gemäss D-AS Nr. 1287580 wird die Herstellung von Isatosäureanhydriden durch Umsetzung von Phthalaimid und Hypohalogeniten beschrieben. Man verwendet das Phthalimid in Gestalt der wässrigen Lösung seines Salzes und setzt das Hypohalogenit zu, bevor mehr als 50% des Phthalimids in der Lösung hydrolysiert sind. Nach Zugabe der Halogenitlösung setzt man in der Reaktionslösung 18000 bis 30000 gcal. frei, bevor man die Lösung auf einen pH-Wert zwischen 5,5 und 9 einstellt. Aus der D-OS Nr. 1770458 ist bekannt, dass gute Ausbeuten an Isatosäureanhydrid nur dann erhalten werden, wenn die eingesetzten Lösungen der Salze der Phthalamidsäure mindestens 5% des Alkalisalzes von Phthalimid enthalten. Wie Beschreibung und Beispiele zeigen, benötigen Phthalamidsäurelösungen, die frei von Phthalimid sind, zum Abbau einen grossen Überschuss von Alkali, der die Ausbeute an Endstoff durch Bildung von Anthranilsäure und Anthranoylanthranilsäure verringert. Die Anwesenheit von Phthalimid oder

dessen Salzen ist nachteilig. Einmal muss Phthalimid aus Phthalsäureanhydrid in einem gesonderten Verfahrensschritt hergestellt werden; zum anderen ist Phthalimid in alkalischer Lösung nur begrenzt haltbar, da es leicht zu Salzen der Phthalamidsäure hydrolysiert.

Es ist aus der D-OS Nr. 2258150 ein Verfahren zur Herstellung von unsubstituiertem oder substituiertem Isatosäureanhydrid durch Umsetzung von phthalamidsaurem Alkalisalz der unsubstituierten oder substituierten Phthalamidsäure mit Hypohalogeniten in wässrigem Medium in Gegenwart von Brom, Jod und/oder Halogenamiden bekannt. Zwar können als Katalysatoren Amidosulfonsäure und ihre Derivate verwendet werden, sie werden aber stets vom Beginn der Reaktion an zugesetzt. In allen Beispielen wird gezeigt, dass die Zugabe des Katalysators, z.B. Amidosulfonsäure, vor Zugabe des Hypohalogenits erfolgt. Die wässrigen Hypohalogenitlösungen enthalten im allgemeinen von 5 bis 15, vorzugsweise von 12 bis 14 Gew.% Hypochlorit und können zusätzlich von 0,01 bis 0,1 mol Alkalihydroxid je Mol Hypohalogenit enthalten. Im Ausgangsgemisch beider Ausgangsstoffe kommen gegebenenfalls Mengen von insgesamt 0,01 bis 0,2 mol Alkalihydroxid (nicht eingerechnet das im Hypochlorit und Ausgangsphthalamidsalz enthaltene Alkali), bezogen auf 1 mol Ausgangsphthalamidsalz, in Frage. Es wird gezeigt, dass das Reaktionsgemisch bis zum Ende der Reaktion alkalisch bleibt und erst bei der Aufarbeitung zur Abtrennung des Endstoffs mit Säure, wie Schwefelsäure, neutralisiert wird (S. 7, 1. Abs.). In den Beispielen wird die Reaktionszeit zwischen Hypohalogenitzugabe und Säurezugabe mit etwa 4 bis 30 s, im einzigen kontinuierlichen Beispiel im Rohrreaktor mit 12 s angegeben. Nach der Säurezugabe liegt der pH-Wert (Beispiele) bei 7 bis 7,5.

Eine entsprechende Umsetzung von unsubstituiertem oder substituiertem Isatosäureanhydrid durch Umsetzung von phthalamidsaurem Alkali mit Hypohalogeniten in Gegenwart von Polymerisationsinhibitoren beschreibt die D-OS Nr. 2346308. Die übrigen Verfahrensmerkmale, insbesondere bezüglich Säurezugabe, Alkalität des Reaktionsgemisches, Reihenfolge bei dem Zusatz der Verfahrenskomponenten, entsprechen den in der D-OS Nr. 2258150 beschriebenen, vorgenannten Merkmalen. Die Verweilzeit zwischen Hypohalogenitzugabe und Säurezugabe beträgt, wie die Beispiele zeigen, von 10 bis 180 s.

Alle diese Verfahren sind im Hinblick auf Ausbeute und Reinheit des Endstoffs unbefriedigend, insbesondere im grosstechnischen, kontinuierlichen Betrieb.

Es wurde nun gefunden, dass man Isatosäureanhydrid der Formel (I)

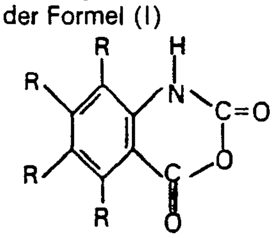

(I)

worin die einzelnen Reste R gleich oder verschieden sein können und jeweils einen Alkylrest mit 1 bis 4 Kohlenstoffatomen, ein Wasserstoffatom oder ein Chloratom bedeuten, durch Mischen einer wässrigen Lösung von phthalamidsaurem und/oder phthalimidsaurem Alkali mit einer wässrigen Hypohalogenitlösung, nachfolgende Umsetzung und rechtzeitiges Einstellen des pH-Wertes auf 5 bis 8, vorteilhaft erhält, wenn

a) Phthalimide der Formel (II)

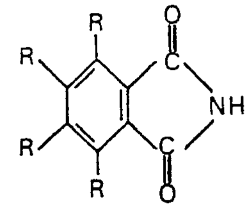

(II)

und/oder Phthalamidsäuren der Formel (III)

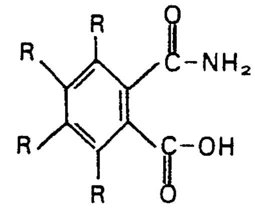

(III)

worin jeweils R die vorgenannte Bedeutung besitzt, in wässriger Alkalilauge löst, wobei 1 bis 1,1 mol Alkalihydroxid je Mol Phthalimid und/oder Phthalamidsäure eingesetzt werden,

b) die so gebildete wässrige Lösung von phthalamidsaurem und/oder phthalimidsaurem Alkali mit 1 bis 2 mol einer wässrigen Lösung von Alkalihypohalogenit je Mol Phthalimid und/oder Phthalamidsäure in einer Mischvorrichtung mischt, wobei man in Stufe a oder b als Katalysatoren Brom, Bromide, Jod, Jodide, Amidosulfonsäure, Alkalisalze der Amidosulfonsäure und/oder Sulfamid zusetzt,

c) das erhaltene Gemisch im ersten Teil eines Reaktionsrohres mit hoher Strömungsgeschwindigkeit von 0,1 bis 10 m/s bei 5 bis 50° C unter weitgehend adiabatischen Bedingungen während 0,1 bis 3,5 s umsetzt, anschliessend

d) den pH-Wert des aus dem ersten Teil des Reaktionsrohres mit hoher Strömungsgeschwindigkeit von 0,1 bis 10 m/s abströmenden Umsetzungsgemisch mit Salzsäure, Schwefelsäure und/oder Phosphorsäure einstellt, und das Umsetzungsgemisch im zweiten Teil des genannten Rohres bei 10 bis 60° C zu Ende umsetzt, und

e) aus dem abströmenden Umsetzungsgemisch in üblicher Weise den Endstoff abtrennt.

Die Umsetzung lässt sich für den Fall der Verwendung von Phthalimid, Phthalamidsäure, Natriumhydroxid, Salzsäure und Natriumhypochlorit durch die folgenden Formeln wiedergeben:

*(Formel auf der nächsten Seite)*

Im Vergleich zu den bekannten Verfahren liefert das Verfahren nach der Erfindung unsubstituiertes oder entsprechend substituiertes Isatosäureanhydrid auf einfacherem und wirtschaftlicherem Wege

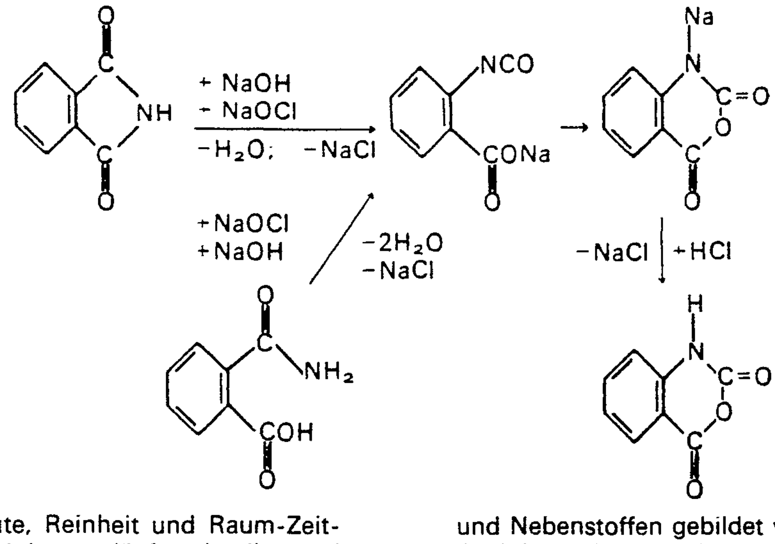


in besserer Ausbeute, Reinheit und Raum-Zeit-Ausbeute. Die Reaktion verläuft schneller und kann somit in wesentlich kleineren Rohrreaktoren durchgeführt werden. Man erhält am Ausgang des Rohrreaktors in der Regel bereits eine Kristallmaische von Isatosäureanhydrid, die zweckmässigerweise in einer Vorlage gesammelt und ohne weitere Nachbehandlung aufgearbeitet werden kann. Der Endstoff fällt in einer gröberen und besser ausgebildeten Kristallform von geringerer Restfeuchte an. Man erhält weniger schmierige, besser filtrierbare und trockenbare Kristalle, die im nachfolgenden Feststofftransport der Trocknungsanlage einen schnelleren und störungsfreieren Betrieb erlauben. Im Vergleich zu dem bekannten Verfahren erspart man sich die Säurezugabe in einem Neutralisationsbad; Reaktion und Neutralisation verlaufen schneller. pH-Schwankungen, die gerade in der üblichen Rührkesselneutralisation zu Zersetzungen des Endstoffs führen, werden vermieden. Der Betrieb der erfindungsgemässen Verfahrens ist deshalb vergleichsweise sicherer und erfordert weniger Betriebs- und Überwachungspersonal. Die Gesamtbetriebszeit, einschliesslich Herstellung des wässrigen Ausgangsgemisches und Aufarbeitung des Reaktionsgemisches, ist bei dem erfindungsgemässen Verfahren kürzer. Alle diese vorteilhaften Ergebnisse sind im Hinblick auf den Stand der Technik überraschend. Im Hinblick auf die genannten Patentschriften war es nicht zu erwarten, dass nach dem Verfahren der Erfindung gute Ergebnisse erzielt werden, obgleich man die Reaktion auch mit phthalamidsaurem Alkali allein und ohne Hydrolyse des Phthalimids nach den in der D-AS Nr. 1287580 beanspruchten Bedingungen durchführt.

Verwendet man Phthalimid als Ausgangsstoff, so ist es im Hinblick auf die D-AS Nr. 1287580 überraschend, dass man in der Regel und bevorzugt das Hypohalogenit zusetzt, nachdem mehr als 50% des Phthalimids in der Lösung hydrolysiert sind; vorteilhaft sind mehr als 95% des Phthalimids vor Zugabe des Hypohalogenits hydrolysiert. Auch musste man erwarten, dass angesichts der sehr kurzen Reaktionszeiten des erfindungsgemässen Verfahrens ein heterogenes Gemisch mit hohen Anteilen an unumgesetztem Ausgangsstoff und Nebenstoffen gebildet wird. Da bei längerer Reaktionszeit perchlorierte Nebenstoffe entstehen, die zu gegebenenfalls explosionsartigen Zersetzungen neigen, ist das erfindungsgemässe Verfahren betriebssicherer.

Im Vergleich zum Verfahren nach DE-B Nr. 1287580 (1) unterscheidet sich das Verfahren der Erfindung darin, dass ein bestimmter Hydrolysegrad nicht erforderlich ist. Gerade dies ist ein vorteilhaftes erfindungsgemässes Merkmal, da in der Praxis die Einstellung und Beibehaltung eines bestimmten Hydrolysegrades schwierig ist, und ständig überwacht werden muss, denn die Ausbeute hängt sehr stark von der Hydrolyse ab, wie aus Beispiel 1 zu ersehen ist. In (1) wird weiterhin gezeigt, dass der Zeitpunkt der Zugabe der Säure für die Ausbeute von erheblicher Bedeutung ist. Es werden jedoch keine Angaben darüber gemacht, wie insbesondere im technischen Betrieb eine rasche pH-Wert-Einstellung erreicht werden kann. Erfolgt nämlich die Säurezugabe wie bei den bekannten Verfahren in einem Rührkessel, so ist auch bei guter Vermischung die Ausbildung eines pH-Gradienten unvermeidlich, insbesondere im technischen Betrieb lassen sich die gewünschten kurzen Verweilzeiten der Reaktionslösung im alkalischen Bereich nicht einstellen. Die in (1) angegebene Verfahrensweise mit Einhaltung eines bestimmten Hydrolysegrades und Säurezugabe, nachdem eine bestimmte Wärmezugabe freigeworden ist, ist im grosstechnischen Betrieb schon aus Sicherheitsgründen kaum durchzuführen. Gerade diese Umsetzung kann sehr leicht ausser Kontrolle geraten, wenn nicht alle Parameter genauestens eingehalten werden. Es ist im Hinblick auf (1) überraschend, dass die Reaktion auch mit vollständig zu phthalamidsaurem Natrium hydrolysiertem Phthalimid ausgeführt werden kann. Durch entsprechende Wahl von Temperatur und Verweilzeit kann aus Phthalimid und Natronlauge eine Lösung hergestellt werden, die nur noch phthalamidsaures Natrium enthält. Die damit erhaltenen Ausbeuten entsprechen Beispiel 1 des erfindungsgemässen Verfahrens.

Wird Phthalimid als Ausgangsstoff verwendet, so ist zweckmässig in Stufe a die Verweilzeit so zu wählen, dass eine vollständige Hydrolyse des

Phthalimids zu Phthalamidsäure erreicht wird, da wesentliche Mengen an Phthalimid in Stufe b das Verfahren nachteilig beeinflussen.

Hierin unterscheidet sich das Verfahren wesentlich vom Verfahren (1), da dort ein bestimmter Hydrolysegrad des Phthalimids vor der Zugabe des Hypohalogenits vorgeschrieben wird.

Weiterhin schreibt (1) vor, dass die Mischung aus Phthalimid/Phthalamidsäure nach Zugabe der Hypohalogenitlösung auf einen pH-Wert von 5,5 bis 9,0 eingestellt wird, wenn 18000 bis 30000 gcal. freigesetzt sind. Wie das erfindungsgemässe Verfahren zeigt, ist die freigesetzte Wärmemenge kein wesentlicher Parameter des Verfahrens, da in Abhängigkeit der Reaktionsbedingungen unterschiedliche Wärmemengen gemessen werden.

Das erfindungsgemässe Verfahren und die erfindungsgemässe Verwendung der Katalysatoren unterscheidet sich wesentlich von dem bekannten Verfahren.

Man erreicht nach dem erfindungsgemässen Verfahren in kürzesten Zeiten eine homogene Vermischung der Säure in der Reaktionslösung und eine Einstellung des gewünschten pH-Wertes, bevor Nebenprodukte gebildet werden können, und deshalb höhere Ausbeuten und reinere Produkte. So erfolgt die Säurezugabe bereits im Reaktor, wobei optimale Ausbeuten erhalten werden, wenn die Verweilzeiten im alkalischen Bereich (Stufe c) extrem kurz sind. Es entfällt somit die umständliche, technisch nicht praktikable Messung der Reaktionsenthalpie vor der Säurezugabe, die komplizierte Regel- und Kontrollapparaturen erfordert, um einen optimalen Prozessablauf zu gewährleisten.

Eine maximale Ausbeute wird erreicht, wenn die Hypohalogenitreaktion nach einer Verweilzeit von 0,5 bis 3,5 s im Reaktionsrohr durch Zugabe von Säure abgestoppt wird.

Die Säurezugabe im Rohrreaktor mit Einstellung einer optimalen Vermischung erlaubt die Einhaltung kürzester Reaktionszeiten in der ersten Stufe, so dass die Kombination aus kurzen Reaktionszeiten und definierter Säurezugabe im Rohrreaktor als überraschend betrachtet werden muss.

Als Ausgangsstoffe verwendet man unsubstituierte oder entsprechend substituierte Phthalimide, Phthalamidsäuren, bzw. phthalamidsaure Alkalisalze (Ausgangsphthalamidsalz) und Alkalihypohalogenite, in der Regel Hypochlorite und Hypobromite in Gestalt von entsprechenden wässrigen, alkalischen Lösungen. Bevorzugte Ausgangsphthalamidsalze sind zweckmässig Kalium- und insbesondere Natriumsalze. Die bevorzugten Ausgangsstoffe tragen vorteilhaft nicht mehr als ein Halogenatom neben der zur Bildung des Anhydridringes benötigten Carboxylgruppe und Carbamidogruppe.

Beispielsweise kommen die Salze folgender Phthalamidsäuren, die Säuren selbst oder entsprechende Phthalimide als Ausgangsstoffe in Betracht: Phthalamid-(2)-säure, 3-Chlorphthalamidsäure, 3,5-Dichlorphthalamidsäure, 3,6-Dichlorphthalamidsäure, 3-Methylphthalamidsäure, 4-Äthylphthalamidsäure, 6-tert.-Butylphthalamidsäure, 4-n-Propyl-5-chlorphthalamidsäure.

Der Ausgangsstoff wird in Stufe a in wässriger Alkalilauge gelöst, zweckmässig Kalilauge und insbesondere Natronlauge. Vorteilhaft gelangen wässrige Lösungen von 5 bis 50, bevorzugt 15 bis 30 Gew.% (bezogen auf die reine Wassermenge) Phthalimid und/oder Phthalamidsäure zur Anwendung, die von 1 bis 1,1, bevorzugt 1 bis 1,02 mol Alkalihydroxid je Mol Phthalimid und/oder 1 bis 1,1, bevorzugt 1 bis 1,02 mol Alkalihydroxid je Mol Phthalamidsäure enthalten. Die Lösung wird zweckmässig bei einer Temperatur von −5 bis +50° C, vorzugsweise von 20 bis 30° C, drucklos oder unter Druck, kontinuierlich durchgeführt. Verwendet man Katalysatoren, z.B. die in der D-OS Nr. 2346308 oder vorteilhaft die in der D-OS 2258150 beschriebenen Katalysatoren, so werden sie zweckmässig schon in Stufe a dem Ausgangsstoff bzw. seiner Lösung zugesetzt. Man kann aber den Katalysator, zweckmässig im Gemisch mit Wasser, auch dem Ausgangsgemisch getrennt oder zusammen mit dem Hypohalogenit zusetzen.

Als Katalysatoren kommen Brom, Jod, Jodide, Amidosulfonsäure, Alkalisalze des Amidosulfonsäure und/oder Sulfamid, im allgemeinen in einer Menge von 0,0001 bis 0,1, vorzugsweise von 0,001 bis 0,01 mol Katalysator je Mol Phthalimid bzw. Phthalamidsäure in Betracht. Anstelle der genannten Stoffe können auch Verbindungen verwendet werden die unter den Reaktionsbedingungen solche Stoffe bilden, z.B. Bromide und Jodide anstelle von Brom oder Jod. Zweckmässig wählt man wasserlösliche Halogenide. Diese Halogenide kommen vorteilhaft in Gestalt ihrer Erdalkali- und insbesondere ihrer Alkalisalze in Frage, z.B. Calciumbromid, Calciumjodid, Magnesiumbromid, Magnesiumjodid, Lithiumbromid, Lithiumjodid und insbesondere Natrium- und Kaliumbromid bzw. Natrium- und Kaliumjodid.

Als Katalysatoren kommen z.B. in Betracht: Amidosulfonsäure und ihre Salze, zweckmässig Alkalisalze wie das Natrium- oder Kaliumsalz, und insbesondere Sulfamid sind bevorzugt, gegebenenfalls im Gemisch miteinander.

Die wässrigen Hypohalogenitlösungen, bevorzugt Hypochchloritlösungen, der Stufe b enthalten vorteilhaft von 5 bis 15, insbesondere 12 bis 14 Gew.% Hypohalogenit, bevorzugt Hypochlorit, und keine wesentlichen Mengen, höchstens bis zu 0,1, insbesondere bis zu 0,01 mol überschüssiges Alkalihydroxid je Mol Phthalimid/Phthalamidsäure. Bevorzugte Alkalihypochlorite sind das Kaliumsalz oder insbesondere Natriumhypochlorit. Die Reaktion wird mit einem Verhältnis von 1 bis 2, vorzugsweise 1 bis 1,1 mol Hypohalogenit je Mol Phthalimid und/oder Phthalamidsäure durchgeführt. Vorteilhaft vermischt man den Ausgangsstoff in seiner wässrigen, alkalischen Lösung vorgenannter Konzentration aus Stufe a mit der Alkalihypohalogenitlösung, bevorzugt Alkalihypochloritlösung, in Stufe b in vorgenanntem Mengenverhältnis in einer Mischungsvorrichtung. Solche Vorrichtungen können Mischzellen, Mischdüsen oder Kammern mit Rührwerken hoher Umdrehungszahl sein. Die Mischung wird in der Regel bei einer Temperatur zwischen 0 und 50° C, vorteilhaft zwischen 25 und 45° C, drucklos oder unter Druck, kontinuierlich durchgeführt.

Die Reaktion wird vorteilhaft in 2 Reaktionsräumen (Stufe c und d) unter weitgehender Vermeidung der Rückmischung in beiden Räumen und weitgehend adiabatisch in der Stufe c und zweckmässig Stufe d durchgeführt. In der Reaktionsstufe c erwärmt die entstehende Reaktionswärme dabei das Umsetzungsgemisch in der Regel auf eine Temperatur zwischen 20 und 50° C. Aus der Mischungsvorrichtung gelangt das Reaktionsgemisch in den Reaktionsraum der ersten Reaktionsstufe (Stufe c), der aus einem vorteilhaft engen Reaktionsrohr besteht, und von dort nach der Umsetzung in den Reaktionsraum der folgenden Stufe (Stufe d), zweckmässig ein weiteres enges Reaktionsrohr; vorheilhaft erfolgen die Stufen c und d in einem einzigen, engen Reaktionsrohr. Mischungsvorrichtung, der Reaktionsraum der ersten Stufe und die Lösungen der Ausgangstoffe brauchen nicht gekühlt zu werden. Am Ende der Stufe c und am Anfang der Stufe d erfolgt die Zuführung der Säure, zweckmässig über Mischdüsen. Ein bevorzugtes Merkmal des Verfahrens nach der Erfindung ist die weitgehende Vermeidung der Rückmischung in Stufe c, ein rascher Entzug des Reaktionsgemisches aus c und seine Zuführung — unter weitgehender Vermeidung der Rückmischung — in die Stufe d. Zweckmässig stellt man durch einen engen Querschnitt des Reaktionsrohres der beiden Stufen und Verwendung entsprechender Transportpumpen eine hohe Strömungsgeschwindigkeit des Reaktionsgemisches ein. Als Pumpen können z.B. Strahl-, Rotations-, Kreiskolben-, Wälzkolben-, Schraubenkolben-, Exzenter-, Flügel-, Kreisel-, Axial-, Propellerpumpen verwendet werden. In einer bevorzugten Ausführungsform des Verfahrens werden die Strömungsgeschwindigkeiten durch Querschnitt und Länge des Reaktionsrohres bestimmt. Vorteilhaft sind z.B. Reaktorquerschnitte von 10 bis 10 000 $mm^2$. Die Strömungsgeschwindigkeiten liegen bei 0,1 bis 10, insbesondere 0,2 bis 3 m/s, vorzugsweise 0,5 bis 1 m/s. Bei diesen Geschwindigkeiten wird «der Ausgangsstoff in der Stufe c» während 0,1 bis 3,5 in der Regel, jedoch während einer Verweilzeit von 0,5 bis 3,5, vorzugsweise von 1 bis 2,7 s weitgehend umgesetzt. Die Verweilzeit der Stufe c ist gleich der Zeit, die das Reaktionsgemisch von der Zugabe von Hypohalogenit bis zur Zugabe der Säure benötigt. Das gebildete Alkalisalz wird, bedingt durch die hohe Strömungsgeschwindigkeit, dem Reaktionsraum der Stufe c sofort entzogen, der folgenden Stufe d zugeführt und dort, im allgemeinen bei einer Verweilzeit von 1 bis 30, vorzugsweise von 2 bis 20 s, mit Säure umgesetzt. Die hohe Strömungsgeschwindigkeit verhindert gleichzeitig weitgehend eine Rückmischung innerhalb der gesamten Umsetzung des Reaktionsgemisches. Insbesondere wird die Rückmischung des gebildeten Endstoffs mit dem Reaktionsgemisch der Stufe c vermieden und damit die Bildung von Nebenprodukten durch Umsetzung des Hypohalogenits bzw. des N-chlorierten Phthalsäuremonoamids mit dem Endstoff und/ oder durch entsprechende Umsetzungen in den Gemischen der Stufen c und d unterdrückt. Die Reaktion wird in Stufe c bei einer Temperatur von 5 bis 50° C, vorzugsweise zwischen 20 und 50° C, insbesondere zwischen 20 und 45° C, in der Stufe d von 10 bis 60° C, vorzugsweise von 30 bis 45° C, drucklos oder unter Druck durchgeführt. Am Ende der Reaktionsfolge wird das Reaktionsgemisch entnommen und kann als Suspension des Isatosäureanhydrids weiterverarbeitet werden, da der Endstoff in ausgezeichneter Reinheit anfällt. Die Isolierung der Endstoffe aus den sauren Lösungen kann durch nachfolgende Filtration vorgenommen werden. Beispielsweise kann das Reaktionsgemisch einem Rührkessel zugeführt und die gebildete Kristallmaische abgesaugt werden. Fällt nach der Reaktion das Isatosäureanhydrid II in Gestalt des Alkalisalzes an, wird es zweckmässig durch nachträgliches Ansäuern in das entsprechende Anhydrid II umgewandelt.

Die Umsetzung des Reaktionsgemisches in der Stufe d wird mit Salzsäure, Schwefelsäure und/ oder Phosphorsäure vorteilhaft mit einer Menge von 1 bis 1,2, insbesondere von 1 bis 1,1 Äquivalenten Säure, bezogen auf 1 mol Ausgangsstoff, durchgeführt. Die Säuren können in konzentrierter Form, im Gemisch miteinander und/oder mit einem Lösungsmittel, insbesondere Wasser, angewendet werden. Bei verdünnten, wässrigen Säuren sind 1- bis 100gew.%ige Säuren, z.B. 3- bis 30gew.%ige Salzsäure, 3- bis 80gew.%ige Schwefelsäure, vorteilhaft. Der pH der Umsetzung in Stufe c beträgt in der Regel von 14 bis 8, vorzugsweise von 12 bis 9, der pH in Stufe d von 8 bis 6, vorzugsweise von 7,5 bis 6,4, insbesondere von 7 bis 6,3. Man regelt die Säurezugabe dergestalt, dass sich im Reaktionsgemisch ein pH im vorgenannten pH-Intervall von 5 bis 8 der Stufe d bald einstellt, vorzugsweise 0 bis 0,1 s nach Zugabe.

Die nach dem Verfahren der Erfindung herstellbaren Verbindungen sind wertvolle Ausgangsstoffe für die Herstellung von Farbstoffen, Pflanzenschutzmitteln und Riechstoffen. Die Isatosäureanhydride können durch Verseifung mit Alkali in die entsprechenden Anthranilsäuren übergeführt werden. Bezüglich der Verwendung wird auf die genannten Patentschriften und „Ullmanns Encyklopädie der technischen Chemie," Bd. 3, S. 465 ff. und Bd. 13, S. 499, verwiesen.

Die in den folgenden Beispielen angeführten Teile bedeuten Gewichtsteile. Sie verhalten sich zu den Volumenteilen wie Kilogramm zu Liter.

*Beispiel 1*

Man verwendet eine Anlage, die aus einer Mischdüse und einem Reaktionsrohr von 4 m Länge und 76 ml Innendurchmesser besteht. 849 Teile flüssiges Phthalimid werden stündlich in einer Mischdüse kontinuierlich in 950 Teilen wässriger, 25gew.%iger Natronlauge und 7900 Teilen Wasser gelöst und kontinuierlich 20 Teile/h

30gew.%ige, wässrige Lösung des Natriumsalzes der Amidosulfonsäure zugeführt. Die gebildete Lösung wird stündlich in der Mischdüse mit 3100 Teilen wässriger Natriumhypochloritlösung (421 Teilen Natriumhypochlorit; 13,6 Gew.% aktives Chlor) bei 25° C gemischt. Die Strömungsgeschwindigkeit im nachfolgenden Reaktionsrohr beträgt 0,77 m/s. In einer Entfernung von 2 m von der Mischdüse befindet sich am Reaktionsrohr eine zweite Mischdüse, aus der stündlich 800 Teile 30gew.%ige Salzsäure in das Reaktionsgemisch gegeben werden. Die Verweilzeit beträgt zwischen den beiden Mischdüsen (Stufe c) 2,6 s, zwischen Zugabe der Säure und Ende des Reaktionsrohres (Stufe d) 2,4 s. Das Reaktionsgemisch wird im ersten Teil des Reaktionsrohres (Stufe c) weitgehend adiabatisch (von 25 bis 30° C) und bei pH 10 umgesetzt, im verbleibenden Reaktionsraum der Stufe d beträgt die Temperatur 35° C das pH 6,8. Das Gemisch wird in einem Rührkessel gesammelt, abfiltriert und der Filterrückstand mit Wasser gewaschen und getrocknet. Man erhält stündlich 922 Teile (98% der Theorie) Isatosäureanhydrid (99,8%) vom Fp.: 235° C (Zersetzung); Raum-Zeit-Ausbeute: 102 Teile/h/l.

*Vergleichsbeispiel* (gemäss DE-A-Nr. 2258150)

Führt man die Umsetzung analog Beispiel 1, aber anstelle des Zumischens von Säure im Reaktor mit einer nachträglichen Neutralisation des Reaktionsgemisches in einem Rührkessel mit stündlich 850 Teilen 30gew.%iger, wässriger Salzsäure durch, so erhält man stündlich aus 849 Teilen Phthalimid, 12722 Teilen Wasser, 1027 Teilen 25gew.%iger Natronlauge, 5,5 Teilen Amidosulfonsäure, 3130 Teilen wässriger Natriumhypochloritlösung (425 Teile Natriumhypochlorit; 13,6 Gew.% aktives Chlor), 800 Teile (85% der Theorie) Isatosäureanhydrid mit einer Reinheit von 97 bis 99%, Fp.: 230° C (Zers.); Raum-Zeit-Ausbeute: 14 Teile/h/l.

*Beispiel 2*

Analog zu Beispiel 1 und unter denselben Bedingungen und Mengenverhältnissen wird aus 1054 Gewichtsteilen 4-Chlorphthalimid das entsprechende Chlorisatosäureanhydrid vom Fp-: 198-204° C in 95%iger Ausbeute erhalten.

## Patentanspruch

Verfahren zur kontinuierlichen Herstellung von Isatosäureanhydrid der Formel (I)

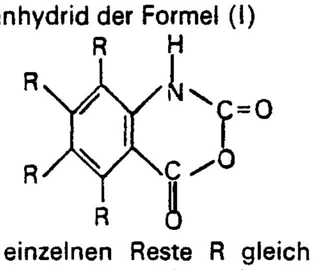

(I)

worin die einzelnen Reste R gleich oder verschieden sein können, und jeweils einen Alkylrest mit 1 bis 4 Kohlenstoffatomen, ein Wasserstoffatom oder ein Chloratom bedeuten, durch Mischen einer wässrigen Lösung von phthalamidsaurem und/oder phthalimidsaurem Alkali mit einer wässrigen Hypohalogenitlösung, nachfolgende Umsetzung und rechtzeitiges Einstellen des pH-Wertes auf 5 bis 8, dadurch gekennzeichnet, dass man

a) Phthalimide der Formel (II)

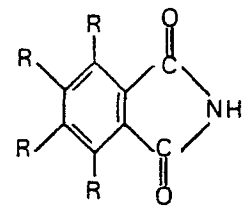

(II)

und/oder Phthalamidsäuren der Formel (III)

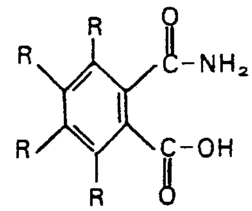

(III)

worin jeweils R die vorgenannte Bedeutung besitzt, in wässriger Alkalilauge löst, wobei 1 bis 1,1 mol Alkalihydroxid je Mol Phthalimid und/oder Phthalamidsäure eingesetzt werden,

b) die so gebildete wässrige Lösung von phthalamidsaurem und/oder phthalimidsaurem Alkali mit 1 bis 2 mol einer wässrigen Lösung von Alkalihypohalogenit je Mol Phthalimid und/oder Phthalamidsäure in einer Mischvorrichtung mischt, wobei man in Stufe a oder b als Katalysatoren Brom, Bromide, Jod, Jodide, Amidosulfonsäure, Alkalisalze der Amidosulfonsäure und/oder Sulfamid zusetzt,

c) das erhaltene Gemisch im ersten Teil eines Reaktionsrohres mit hoher Strömungsgeschwindigkeit von 0,1 bis 10 m/s bei 5 bis 50° C unter weitgehend adiabatischen Bedingungen während 0,1 bis 3,5 s umsetzt, anschliessend

d) den pH-Wert des aus dem ersten Teil des Reaktionsrohres mit hoher Strömungsgeschwindigkeit von 0,1 bis 10 m/s abströmenden Umsetzungsgemisches mit Salzsäure, Schwefelsäure und/oder Phosphorsäure einstellt, und das Umsetzungsgemisch im zweiten Teil des genannten Rohres bei 10 bis 60° C zu Ende umsetzt, und

e) aus dem abströmenden Umsetzungsgemisch in üblicher Weise den Endstoff abtrennt.

## Revendication

Procédé de préparation en continu de l'anhydride isatoïque de la formule (I)

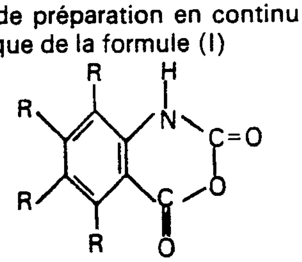

(I)

dans laquelle les divers restes R, qui peuvent être identiques ou différents, désignent chacun un radical alcoyle en $C_1$ à $C_4$, un atome d'hydrogène ou un atome de chlore, par mélange d'une solution aqueuse d'un sel de métal alcalin de l'acide phtalamidique et(ou) du phtalimide et d'une solution aqueuse d'un hypohalogénite, suivi de la réaction et de l'ajustement, au moment voulu, du pH entre 5 et 8, caractérisé en ce que:

a) on dissout des phtalimides de la formule (II)

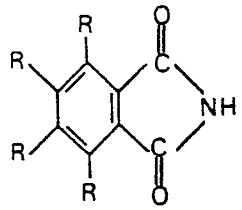

(II)

et(ou) des acides phtalamidiques de la formule (III)

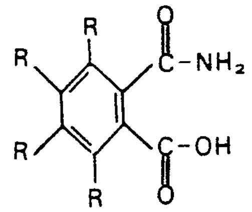

(III)

dans lesquelles les restes R possèdent la signification définie, dans une lessive alcaline aqueuse, contenant 1 à 1,1 mol d'hydroxyde de métal alcalin par mole de phtalimide et(ou) d'acide phtalamidique;

b) on ajoute à la solution aqueuse de sels d'alcali d'acide phtalamidique et(ou) de phtalimide ainsi formée, dans une installation de mélange, 1 à 2 mol d'une solution aqueuse d'un hypohalogénite de métal alcalin par mole de phtalimide et(ou) d'acide phtalamidique avec addition soit dans le stade a, soit dans le stade b, de brome, d'un bromure, d'iode, d'un iodure, d'acide amidosulfonique, d'un sel de métal alcalin d'acide amidosulfonique et(ou) d'un sulfamide comme catalyseur;

c) on fait réagir le mélange formé pendant 0,1 à 3,5 s, dans des conditions sensiblement adiabatiques, dans la première partie d'un réacteur tubulaire entre 5 et 50° C avec une vitesse d'écoulement élevée de 0,1 à 10 m/s;

d) on ajuste le pH du mélange réactionnel, quittant la première partie du réacteur tubulaire avec une vitesse élevée de 0,1 à 10 m/s, à l'aide d'acide chlorhydrique, d'acide sulfurique et(ou) d'acide phosphorique, puis on complète la réaction dans la deuxième partie du réacteur tubulaire entre 10 et 60° C;

e) on sépare du mélange réactionnel quittant le réacteur le produit final d'une manière usuelle.

## Claim

A process for the continuous preparation of isatoic anhydride of the formula I

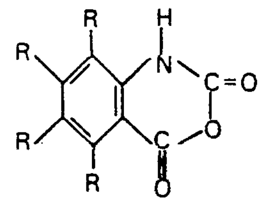

(I)

where the individual radicals R may be identical or different and each denotes alkyl of 1 to 4 carbon atoms, hydrogen or chlorine, by mixing an aqueous solution of an alkali metal phthalamate and/or an alkali metal phthalimidate with an aqueous hypohalite solution, subsequent reaction and timely adjustment of the pH to 5 to 8, characterized in that

a) a phthalimide of the formula II

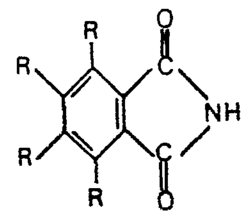

(II)

and/or a phthalamic acid of the formula III

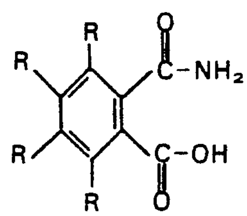

(III)

where each R has the above meaning, is dissolved in an aqueous alkali metal hydroxide solution, from 1 to 1.1 mol of alkali metal hydroxide being used per mole of phthalimide and/or per mole of phthalamic acid,

b) the resulting aqueous solution of alkali metal phthalamate and/or alkali metal phthalimidate is mixed with 1 to 2 mol of an aqueous solution of an alkali metal hypohalite per mole of phthalimide and/or per mole of phthalamic acid in a mixing apparatus, bromine, a bromide, iodine, an iodide, amidosulfonic acid, an alkali metal salt of amidosulfonic acid and/or sulfamide being added as catalyst in stage a or b,

c) the resulting mixture is reacted in the first part of a reaction tube, at a high flow rate of from 0.1 to 10 m/s, under substantially adiabatic conditions at from 5 to 50° C for from 0.1 to 3.5 s, thereafter

d) the pH of the reaction mixture issuing at a high flow rate of from 0.1 to 10 m/s from the first part of the reaction tube is adjusted with hydrochloric acid, sulfuric acid and/or phosphoric acid, and the reaction mixture is reacted to completion in the second part of the said tube at from 10 to 60° C, and

e) the end product is isolated from the issuing reaction mixture in a conventional manner.